# EUROPÄISCHE PATENTSCHRIFT

(11) **EP 2 048 946 B1**
(45) Veröffentlichungstag und Bekanntmachung des Hinweises auf die Patenterteilung: **06.01.2016**
(21) Anmeldenummer: 07788222.3
(22) Anmeldetag: 03.08.2007
(51) Int. Cl.: A01N 43/56, A01N 43/80

(54) **NICHT-WÄSSRIGE WIRKSTOFFKONZENTRATE MIT HERBIZIDER WIRKUNG**
NON-AQUEOUS ACTIVE INGREDIENT CONCENTRATES HAVING AN HERBICIDAL EFFECT
CONCENTRES NON AQUEUX DE PRINCIPES ACTIFS A EFFET HERBICIDE

(30) Priorität: 04.08.2006 EP 06118445
(43) Veröffentlichungstag der Anmeldung: 22.04.2009
(73) Patentinhaber: BASF SE, 67056 Ludwigshafen (DE)
(72) Erfinder: KRAPP, Michael, 67122 Altrip (DE); BERGHAUS, Rainer, 67346 Speyer (DE); BRATZ, Matthias, 67133 Maxdorf (DE); KIBLER, Elmar, 67454 Hassloch (DE); VANTIEGHEM, Herve R., 76297 Stutensee (DE)
(86) Internationale Anmeldenummer: PCT/EP2007/058091
(87) Internationale Veröffentlichungsnummer: WO 2008/015279

(56) Entgegenhaltungen:
- EP-A- 1 023 833
- WO-A-99/65314
- WO-A-2007/060146

## Beschreibung

Die vorliegende Erfindung betrifft Wirkstoffkonzentrate mit herbizider Wirkung, enthaltend
a) mindestens eine 4-Benzoyl-substituierte Pyrazolverbindung der Formel I 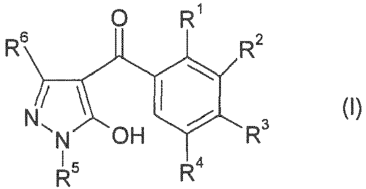 worin
   R¹ und R⁵ jeweils Methyl bedeuten, R² für 4,5-Dihydroisoxazol-3-yl steht, R³ Methylsulfonyl bedeutet und R⁴ und R⁶ Wasserstoff bedeutet, oder eines seiner landwirtschaftlich verwendbaren Salze; und
b) 2-Chlor-N-(2,4-dimethyl-3-thienyl)-N-(2-methoxy-1-methylethyl)acetamid (Dimethenamid).

Die Erfindung betrifft auch die Verwendung derartiger Wirkstoffkonzentrate zur Bekämpfung von unerwünschtem Pflanzenwuchs, insbesondere zur Bekämpfung von grasartigen Schadpflanzen.

Zur effizienten und rentablen Durchführung einer technisierten Landwirtschaft und zur Sicherung einer gleichbleibenden Produktqualität sind Reinkulturen der landwirtschaftlich interessanten Nutzpflanzen erforderlich. Die selektive Empfindlichkeit unterschiedlicher Pflanzengruppen gegenüber bestimmten Stoffwechselinhibitoren oder anderen Zellgiften kann zur gezielten Bekämpfung von unerwünschtem Fremdpflanzenwuchs (Schadpflanzenbewuchs) auf den landwirtschaftlichen Nutzflächen genutzt werden. Hierbei ist es grundsätzlich wünschenswert, sowohl die absolute Wirksamkeit als auch die Spezifität der eingesetzten Wirkstoffe (Herbizide) gegen Schadpflanzen zu steigern.

Eine Erhöhung der Spezifität sowie in gewissen Grenzen der absoluten Wirksamkeit kann durch Verwendung von Kombinationen mehrerer spezifischer Wirkstoffe, die an unterschiedlichen Punkten des Stoffwechsels der Zielpflanzen angreifen, erreicht werden. Man spricht hierbei von Synergismus (gelegentlich auch von überadditiven Effekte), wenn die Wirkung der Kombination deutlich über der Summe der Einzelwirkungen liegt.

Die herbizide Wirkung von 4-Benzoyl-substituierten Pyrazolverbindungen der Formel I ist aus WO 96/26206 und WO 98/31681 bekannt.

Die herbizide Wirkung von 2-Chlor-N-(2,4-dimethyl-3-thienyl)-N-(2-methoxy-1-methyl-ethyl)acetamid, das auch als Dimethenamid bezeichnet wird, ist aus GB 2,114,566 bekannt. Bei Dimethenamid handelt es sich aufgrund des Vorliegens zweier chiraler Elemente (chirale Achse entlang der Bindung zwischen der 3-Position des Thiofenrings und dem N-Atom der Amidgruppe sowie ein Asymmetriezentrum am Kohlenstoff 1 der 2-Methoxy-1-methylethyl-Gruppe) um ein Gemisch von vier Stereoisomeren. Die Stereoisomere von Dimethenamid, die bezüglich des asymmetrischen Kohlenstoffatoms der 2-Methoxy-1-methylethyl-Gruppe S-Konfiguration aufweisen, werden auch als S-Isomer bzw. als Dimethenamid-P bezeichnet.

Aus der WO 99/65314 ist bekannt, dass die gemeinsame Applikation von 4-Benzoylsubstiuierten Pyrazolverbindungen der Formel I mit Dimethenamid zu einer im Vergleich zur Applikation der Einzelverbindungen erhöhten herbiziden Wirkung führt. Formulierungen, die beide Wirkstoffe enthalten, werden dort nicht beschrieben.

Nicht zuletzt aus Praktikabilitätsgründen sind Formulierungen wünschenswert, die sowohl eine 4-Benzoyl-substituierte Pyrazolverbindung der Formel I als auch Dimethenamid in möglichst konzentrierter Form enthalten. Hierbei ist eine Reihe von Problemen zu lösen, insbesondere wenn die Formulierung die Wirkstoffe in konzentrierter Form enthält. Da derartige Wirkstoffkonzentrate vor ihrer Applikation in der Regel mit Wasser verdünnt werden, muss gewährleistet sein, dass sich die Konzentrate problemlos mit Wasser verdünnen lassen und in der erhaltenen wässrigen Verdünnung die Wirkstoffe in möglichst gleichmäßig verteilter Form vorliegen. Häufig neigen jedoch gerade konzentrierte Wirkstoffformulierungen (Wirkstoffkonzentrate) bei längerer Lagerung zur Phasenseparation und/oder zur Abscheidung von Feststoffen. Hierdurch kommt es dann beim Verdünnen mit Wasser zu einer ungleichmäßigen Verteilung der Wirkstoffe in der wässrigen Verdünnung und/oder zu Ungenauigkeiten bei der Dosierung der Wirkstoffe, wodurch häufig der gewünschte überadditive Effekt verloren geht.

Der vorliegenden Erfindung liegt somit die Aufgabe zu Grunde, eine Formulierung für ein Gemisch von 4-Benzoyl-substituierten Pyrazolverbindungen der Formel I, wie eingangs definiert, mit Dimethenamid bereitzustellen, das die beiden Wirkstoffe in möglichst konzentrierter Form enthält.

Gemäß einem ersten Gegenstand der vorliegenden Erfindung werden diese und weitere Aufgaben durch ein nicht-wässriges Wirkstoffkonzentrat gelöst, welches
a) 10 bis 100 g/L insbesondere 20 bis 50 g/L mindestens einer 4-Benzoyl-substituierten Pyrazolverbindung der Formel I wie zuvor definiert oder eines seiner landwirtschaftlich verwendbaren Salze,
b) 200 bis 700 g/L insbesondere 400 bis 600 g/L 2-Chlor-N-(2,4-dimethyl-3-thienyl)-N-(2-methoxy-1-methylethyl)acetamid, und
c) 10 bis 200 g/L insbesondere 20 bis 100 g/L mindestens einer oberflächenaktiven Substanz S, die ausgewählt ist unter einem Gemisch wenigstens einer anionischen oberflächenaktiven Substanz bzw. Verbindung und wenigstens einer nichtionischen oberflächenaktiven Substanz bzw. Verbindung,
wobei die anionische oberflächenaktive Substanz ausgewählt ist unter Verbindungen, die wenigstens eine SO₃-Gruppe oder eine PO₄-Gruppe und wenigstens einen aliphatischen Kohlenwasserstoffrest mit 8 bis 22 C-Atomen oder einen araliphatischen Kohlenwasserstoffrest mit 10 bis 24 C-Atomen aufweisen, und die nichtionische oberflächenaktive Substanz als Hauptbestandteil wenigstens eine Poly-C₂-C₃-alkylenglykolether-Verbindung umfasst,
wobei die Bestandteile a), b) und c) in einem organischen Lösungsmittelgemisch gelöst vorliegen, das zu wenigstens 95 Gew.-%, insbesondere wenigstens 99 Gew.-%, bezogen auf das Lösungsmittelgemisch, aus
d1) wenigstens einem aprotisch polaren organischen Lösungsmittel, das eine Mischbarkeit mit Wasser bei 25 °C und 1 bar von wenigstens 50 g/l aufweist, und das ausgewählt ist unter Dimethylsulfoxid, Sulfolan, den Amiden, N-C₁-C₄-Alkyl-amiden und N,N-Di-(C₁-C₄-alkyl)amiden aliphatischer Monocarbonsäuren mit 1 bis 12 C-Atomen, N-C₁-C₄-Alkyllactamen und deren Gemischen;
d2) wenigstens einem organischen Lösungsmittel, das bei 25 °C und 1 bar in Wasser eine Löslichkeit von weniger als 5 g/l, insbesondere weniger als 1 g/l aufweist, besteht.

Derartige nicht-wässrige Wirkstoffkonzentrate sind besonders lagerstabil und lassen sich problemlos mit Wasser auf die gewünschte Anwendungskonzentration verdünnen. Die unter Verwendung der nicht-wässerigen Wirkstoffkonzentrate hergestellten wässerigen Wirkstoffaufbereitungen weisen zudem eine geringe Schaumbildung (bestimmt nach Ross-Miles) auf. Die erhaltenen wässerigen Wirkstoffaufbereitungen sind zudem besonders stabil gegenüber Entmischung (bestimmt nach CIPAC MT). Zudem beobachtet man überraschenderweise bei Applikation der nicht-wässrigen, erfindungsgemäßen Wirkstoffkonzentrate eine erhöhte herbizide Wirkung im Vergleich zur gemeinsamen Applikation getrennter Wirkstoffformulierungen von Wirkstoffen der Formel I und Dimethenamid.

Gemäß einem zweiten Gegenstand der vorliegenden Erfindung werden die vorgenannten Aufgaben auch durch ein wässriges Wirkstoffkonzentrat gelöst, das
a) 10 bis 100g/L, insbesondere 20 bis 50 g/L mindestens einer 4-Benzoyl-substituierten Pyrazolverbindung der Formel I, wie zuvor definiert,
b) 200 bis 700 g/L, insbesondere 400 bis 600 g/L 2-Chlor-N-(2,4-dimethyl-3-thienyl)-N-(2-methoxy-1-methylethyl)acetamid, und
c) 10 bis 200 g/L, insbesondere 20 bis 100 g/L mindestens einer oberflächenaktiven Substanz S, die ausgewählt ist unter Gemischen wenigstens einer nichtionischen, oberflächenaktiven Substanz mit anionischen oberflächenaktiven Substanzen,
enthält, wobei die Bestandteile a) und b) in einem wässrigen Verdünnungsmittel in disperser Form vorliegen.

Auch die erfindungsgemäßen wässrigen Wirkstoffzusammensetzungen sind über einen langen Zeitraum auch bei erhöhter Temperatur lagerstabil und lassen sich problemlos mit Wasser auf die gewünschte Anwendungskonzentration verdünnen. Sie zeichnen sich zudem durch einen geringen Gehalt an flüchtigen organischen Kohlenwasserstoffen aus.

Hier und im Folgenden bedeuten Alkyl und die Alkylteile in Alkylcarbonyl, Alkoxy, Alkylthio und Alkylphenyl, lineare oder verzweigte, gesättigte Kohlenwasserstoffreste. Alkenyl steht dementsprechend für lineare oder verzweigte Kohlenwasserstoffreste, die einfach ungesättigt sind. Halogenalkyl sowie die Halogenalkylteile in Halogenalkoxy stehen für lineare oder verzweigte Alkylreste, worin 1 oder mehrere, z. B. 1, 2, 3, 4, 5 oder auch alle Wasserstoffatome durch Halogen, insbesondere durch Chlor oder Fluor ersetzt sind. Phenylalkyl steht für einen Phenylrest, der mit dem Rest des Moleküls über eine Alkylgruppe verbunden ist. Cycloalkyl steht für cyclische, gesättigte Kohlenwasserstoffreste. Das Präfix Cₙ-Cₘ gibt jeweils die Anzahl möglicher Kohlenstoffatome an.

Beispiele sind Alkyl sind C₁-C₄-Alkyl wie Methyl, Ethyl, Propyl, 1-Methylethyl, Butyl, 1-Methylpropyl, 2-Methylpropyl und 1,1-Dimethylethyl, weiterhin C₁-C₆-Alkyl, das neben den für C₁-C₄-Alkyl genannten Resten auch Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl zählen, sowie längerkettige Alkylreste wie n-Heptyl, n-Octyl, n-Nonyl, Isononyl, 2-Ethylhexyl, n-Decyl, Isodecyl, 2-Propylheptyl, Dodecyl, Tridecyl, Isotridecyl, Pentadecyl, Lauryl, Myristyl, Palmityl, Stearyl, Behenyl und dergleichen.

Alkylcarbonyl steht für einen über eine Carbonylgruppe gebundenen Alkylrest, wie zuvor genannt.

Alkoxy steht für einen über Sauerstoff gebunden Alkylrest, wie zuvor definiert, insbesondere für C₁-C₄-Alkoxy wie Methoxy, Ethoxy, Propoxy, 1-Methylethoxy, Butoxy, 1-Methylpropoxy, 2-Methylpropoxy und 1,1-Dimethylethoxy.

Haloalkyl steht für einen Alkylrest, wie vorstehend definiert, worin ein oder mehrere, z. B. 1, 2, 3, 4 oder 5 oder alle Wasserstoffatome durch Halogen, insbesondere durch Fluor oder Chlor ersetzt sind. Beispiele sind Fluormethyl, Chlormethyl, Trifluormethyl, Difluorethyl, 2,2,2-Trifluorethyl, Pentafluorethyl, 2-Fluor-1-methylethyl, 2,2,2-Trifluor-1-methylethyl, etc.

Cycloalkyl steht für einen cyclischen, gesättigten Kohlenwasserstoff-Rest wie beispielsweise Cyclopentyl, Cyclohexyl, Cycloheptyl.

Phenylalkyl steht für einen über eine Alkylgruppe gebundenen Phenylrest, wie beispielsweise Benzyl, 1- oder 2-Phenylethyl.

5-gliedrige heterocyclische Reste sind gesättigte, teilgesättigte oder aromatische Cyclen, die 5-Ringatome (Ringglieder) aufweisen und die neben den Kohlenstoffatomen als Ringglieder ein oder mehrere, z. B. 1, 2, 3 oder 4 Heteroatome, insbesondere 1 oder 2 Heteroatome als Ringglieder aufweisen, wobei die Heteroatome vorzugsweise unter O, S und N ausgewählt sind. Beispiele hierfür sind 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-lsothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl, Pyrrol-1-yl, Pyrazol-1-yl, Imidazol-1-yl, 1,2,3-Triazol-1-yl und 1,2,4-Triazol-1-yl.

Erfindungsgemäß enthalten die Wirkstoffkonzentrate eine Verbindung der Formel I, worin R¹ Methyl, R² 4,5-Dihydro-isoxazol-3-yl, R³ Methylsulfonyl, R⁴ Wasserstoff, R⁵ Methyl und R⁶ Wasserstoff bedeuten, d. h. die Komponente a) ist 4-[2-Methyl-3-(4,5-dihydroisoxazol-3-yl)-4-methylsulfonyl-benzoyl]-1-methyl-5-hydroxy-1H-pyrazol (Common name: Topramezone).

Die Komponente b) der erfindungsgemäßen Wirkstoffkonzentrate kann als racemisches Diastereomerengemisch eingesetzt werden oder in Form eines Gemisches, das eines, zwei oder drei der vier Diastereomere in angereicherter Form enthält. Insbesondere werden als Komponente das sogenannte "S-Isomer" von Dimethenamid, d. h. Dimethenamid P, sowie Gemische der Stereoisomere dieser Verbindung bevorzugt, welche überwiegend aus 1S-2-Chlor-N-(2,4-dimethyl-3-thienyl)-N-(2-methoxy-1-methyl-ethyl)acetamid bestehen. Hierunter wird als Komponente b) insbesondere reines Dimethenamid-P oder Gemische der Stereoisomere dieser Verbindung bevorzugt, in denen das Verhältnis von "S-Isomer" (Dimethenamid P, d. h. Stereoisomere des Dimethenamids mit S-Konfiguration am asymmetrischen Kohlenstoffatom der 2-Methoxy-1-methylethyl-Gruppe) zu "R-Isomer" (Stereoisomere mit R-Konfiguration am asymmetrischen Kohlenstoffatom dieser Gruppe) wenigstens 8 : 2 und insbesondere wenigstens 9 : 1 beträgt.

Die erfindungsgemäßen nicht-wässrigen Wirkstoffkonzentrate enthalten neben den Wirkstoffen der Formel I und Dimethenamid bzw. Dimethenamid P wenigstens eine oberflächenaktive Substanz, die zur Stabilisierung der beim Verdünnen mit Wasser entstehenden Wirkstoff-Lösungsmittel-Tröpfchen in dem wässrigen Verdünnungsmittel geeignet sind. Erfindungsgemäß handelt es sich hierbei um ein Gemisch aus wenigstens einer anionischen oberflächenaktiven Substanz und wenigstens einer nichtionischen oberflächenaktiven Substanz. Das Gewichtsverhältnis der wenigstens einen anionischen oberflächenaktiven Substanz zu der wenigstens einen nichtionischen oberflächenaktiven Substanz liegt typischerweise im Bereich von 1 : 10 bis 10 : 1.

Als anionische oberflächenaktive Substanzen sind grundsätzlich alle anionischen ober-flächenaktiven Substanzen geeignet, wie sie typischerweise zur Stabilisierung von wässrigen o/w-Emulsionen eingesetzt werden. Hierbei handelt es sich in der Regel um organische Verbindungen, die einen hydrophoben Rest, typischerweise einen Kohlenwasserstoffrest mit 6 bis 40, häufig mit 6 bis 30 und insbesondere mit 8 bis 22 Kohlenstoffatomen, und wenigstens eine funktionelle Gruppe, die im Wässrigen in anionischer Form vorliegt, beispielsweise eine Carboxylat-, Sulfonat-, Sulfat-, Phosphonat-, Phosphat-, Hydrogenphosphat-, oder Dihydrogenphosphat-Gruppe, aufweisen. Gegebenenfalls weisen die anionischen oberflächenaktiven Substanzen zusätzlich eine Poly-C₂-C₃-Alkylenether-Gruppe, insbesondere eine Polyethylenoxid-Gruppe mit 1 bis 50, insbesondere 2 bis 30 C₂-C₃-Alkylenoxid-Wiederholungseinheiten, insbesondere Ethylenoxid-Wiederholungseinheiten auf.

Erfindungsgemäß weisen die anionischen oberflächenaktiven Substanzen wenigstens eine SO₃-Gruppe (Sulfat und/oder Sulfonat) oder eine PO₄-Gruppe (Phosphat-Gruppe) und wenigstens einen aliphatischen Kohlenwasserstoffrest mit 8 bis 22 C-Atomen oder einen araliphatischen Kohlenwasserstoffrest mit 10 bis 26 C-Atomen auf. Derartige anionische oberflächenaktive Substanzen werden typischerweise in Form ihrer Alkalimetall-, Erdalkalimetall- oder Ammoniumsalze, insbesondere in Form ihrer Natrium-, Kalium-, Kalzium- oder Ammoniumsalze verwendet. Der Begriff aliphatisch soll hier und im Folgenden Alkyl, Alkenyl und Alkadienyl umfassen und steht vorzugsweise für Alkyl. Der Begriff Aralkyl steht für einen aromatischen Kohlenwasserstoffrest wie Phenyl oder Naphthyl, und vorzugsweise für Phenyl, der ein oder mehrere, insbesondere eine Alkylgruppe aufweist.

Beispiele hierfür sind:
c.1. C₈-C₂₂-Alkylsulfonate wie Laurylsulfonat und Isotridecylsulfonat;
c.2. C₈-C₂₂-Alkylsulfate wie Laurylsulfat, Isotridecylsulfat, Cetylsulfat und Stearylsulfat;
c.3. Aryl und C₄-C₂₀-Alkylarylsulfonate wie Naphthalinsulfonat, Dibutylnaphthalinsulfonat, Dodecyldiphenylethersulfonat, Cumolsulfonat, Nonylbenzolsulfonat, Dodecylbenzolsulfonat, Isotridecylbenzolsulfonat;
c.4. Sulfate und Sulfonate von Fettsäuren mit vorzugsweise 8 bis 22 C-Atomen und von Fettsäureestern, z.B. Sulfate und Sulfonate von Mono-, Di- und Triglyceriden und von C₁-C₁₈-Alkyl C₈-C₂₂-alkanoaten;
c.5. Sulfate ethoxylierter C₈-C₂₂-Alkanole, beispielsweise die Sulfate von ethoxyliertem Laurylalkohol, von ethoxyliertem Isotridecanol, von ethoxyliertem C₁₆-C₁₈-Alkanolgemischen, von ethoxyliertem Stearylalkohol, etc.;
c.6. Sulfate ethoxylierter Hydroxyaromaten, insbesondere Sulfate ethoxylierter Phenole, z. B. Sulfate ethoxylierter C₄-C₂₂-Alkylphenole, beispielsweise die Sulfate von ethoxyliertem Octylphenol, von ethoxyliertem Nonylphenol, von ethoxyliertem Dodecylphenol und von ethoxyliertem Tridecylphenol sowie die Sulfate ethoxylierter Mono-, Di- oder Tristyrylphenole;
c.7. Mono- und Diester der Phosphorsäure, einschließlich deren Mischungen mit Triestern der Phosphorsäure, insbesondere die Ester mit C₈-C₂₂-Alkanolen, ethoxylierten C₈-C₂₂-Alkanolen, mit C₄-C₂₂-Alkylphenolen, mit ethoxylierten C₄-C₂₂-Alkylphenolen, mit Mono-, Di- oder Tristyrylphenolen sowie mit ethoxylierten Mono-, Di- oder Tristyrylphenolen sowie Gemische davon;
c.8. Mono- und Di-C₄-C₂₂-Alkylester der Sulfobernsteinsäure wie Dihexylsulfosuccinat, Dioctylsulfosuccinat, und Bis-2-ethylhexylsulfosuccinat; sowie
c.9. Kondensationsprodukte von Naphthalinsulfonsäure oder Phenolsulfonsäure mit Formaldehyd und gegebenenfalls Harnstoff.

Bevorzugte anionische oberflächenaktive Substanzen für die erfindungsgemäßen nicht-wässrigen Wirkstoffkonzentrate sind solche der Gruppen c.1., c.2., c.3., c.5., c.6. und c.7., insbesondere solche, die einen aliphatischen Kohlenwasserstoffrest, d. h. einen Alkyl-, Alkenyl- oder Alkadienyl-Rest, mit 8 bis 22 C-Atomen und/oder einen C₄-C₂₂-Alkylphenyl-Rest aufweisen. In einer besonders bevorzugten Ausführungsform der vorliegenden Erfindung umfasst die anionische oberflächenaktive Substanz wenigstens eine oberflächenaktive Substanz aus den Gruppen c.2. oder c.3. und wenigstens eine weitere oberflächenaktive Substanz aus der Gruppe c.7.

Geeignete nichtionische oberflächenaktive Substanzen sind solche mit einer Poly-C₂-C₃-alkylenglykolether-Gruppe, die im Folgenden auch als Poly-C₂-C₃-alkoxylate oder als Poly-C₂-C₃-alkylenglykolether bezeichnet werden, sowie Polyethylenoxid-Polypropylenoxid-Copolymere, insbesondere Blockcopolymere. Die Begriffe Poly-(ethylenglykol-co-propylenglykol) und Poly(ethoxylat-co-propoxylat) werden im Folgenden synonym verwendet und stehen für Verbindungen mit einer Poly-C₂-C₃-alkylenglykolether-Gruppe, die aus Ethylenoxid und Propylenoxid-Wiederholungseinheiten aufgebaut sind.

Beispiele für geeignete oberflächenaktive Substanzen aus der Gruppe der Poly-C₂-C₃-alkoxylate sind insbesondere
c.10. Poly-C₂-C₃-alkylenglykolalkylether, insbesondere Polyethylenglykolalkylether und Poly(ethylenglykol-co-propylenglykol)-Alkylether von linearen oder verzweigten C₈-C₂₂-Alkanolen, insbesondere Polyethoxylate und Poly(ethoxylat-co-propoxy-late) von Fettalkoholen und von Oxoalkoholen, beispielsweise Polyethoxylate des Laurylalkohols, Poly(ethoxylat-co-propoxylate) des Laurylalkohols, Polyethoxylate des Isotridecanols, Poly(ethoxylat-co-propoxylate) des Isotridecanols, Polyethoxylate des Cetylalkohols, Poly(ethoxylat-co-propoxylate) des Cetylalkohols, Polyethoxylate des Stearylalkohols und Poly(ethoxylat-co-propoxylate) des Stearylalkohols sowie die entsprechenden C₁-C₄-Alkylether, insbesondere die Methylether und die C₁-C₄-Alkanoate dieser Verbindungen;
c.11. Poly-C₂-C₃-alkylenglykolarylether, insbesondere Polyethoxylate und Poly-(ethoxylat-co-propoxylate) von Hydroxyaromaten, z. B. von C₁-C₂₂-Alkylphenolen wie beispielsweise die Polyethoxylate und Poly(ethoxylat-co-propoxylate) von Nonylphenol, Decylphenol, Isodecylphenol, Dodecylphenol, Isotridecylphenol, von Mono-, Di- oder Tristyrylphenol und deren Mischungen sowie die C₁-C₄-Alkylether, insbesondere die Methylether, und die C₁-C₄-Alkanoate der vorgenannten Ethoxylate und Poly(ethoxylat-co-propoxylate);
c.12. Poly-C₂-C₃-alkoxylate, insbesondere Polyethoxylate von C₈-C₂₂-Alkylglucosiden und Poly-C₂-C₃-alkoxylate, insbesondere Polyethoxylate von C₈-C₂₂-Alkylpolyglucosiden;
c.13. Poly-C₂-C₃-alkoxylate, insbesondere Polyethoxylate und Poly(ethoxylat-copropoxylate) von Fettaminen, insbesondere Polyethoxylate und Poly(ethoxylatco-propoxylate) von Stearylamin, Talgfettamin, Oleylamin und Kokosfettamin;
c.14. Poly-C₂-C₃-alkoxylate, insbesondere Polyethoxylate von Fettsäuren, beispielsweise Polyethoxylate der Stearinsäure, der Laurinsäure, der Ölsäure, der Myristinsäure, von Gemischen der vorgenannten Fettsäuren;
c.15. Polyethoxylierte Fette und Öle, beispielsweise Polyethoxylate von Kokosöl, Palmkernöl, Talgfettöl, Palmöl, Rapsöl, Sonnenblumenöl, oder Rizinusöl; sowie
c.16. Poly-C₂-C₃-alkoxylate, insbesondere Polyethoxylate von Sorbitanfettsäureestern, beispielsweise Polyethoxylate von Sorbitanmono-, di- oder trioleat und deren Gemische.

In den vorgenannten Polyethoxylaten liegt der Ethoxylierungsgrad (mittlere Anzahl der von Ethylenoxid abgeleiteten Wiederholungseinheiten im Molekül) typischerweise im Bereich von 2 bis 100, insbesondere im Bereich von 3 bis 50 und speziell im Bereich von 5 bis 40. In den (Poly)Ethoxylat-co-propoxylaten beträgt die mittlere Anzahl an Wiederholungseinheiten, die von Ethylenoxid abgeleitet sind, in der Regel 1 bis 50, insbesondere 2 bis 40 und speziell 3 bis 30 und die mittlere Anzahl an Wiederholungseinheiten, die von Propylenoxid abgeleitet sind, 1 bis 50, insbesondere 2 bis 40 und speziell 2 bis 30.

Zu den bevorzugten nichtionischen oberflächenaktiven Substanzen zählen auch Copolymere, insbesondere Blockcopolymere von Ethylenoxid und Propylenoxid (nachfolgend EO/PO-Copolymere genannt). Hierunter versteht man oligomere oder polymere Polyetherverbindungen, die überwiegend, d.h. zu wenigstens 90 Gew.-% aus Wiederholungseinheiten EO (CH₂-CH₂-O) und PO (= CH₂-CH(CH₃)-O) aufgebaut sind. Hierunter sind Ethylenoxid-Propylenoxid-Blockcopolymere bevorzugt, wobei die Anzahl der PO-Blöcke und der EO-Blöcke vorzugsweise 2 oder insbesondere 3 beträgt. Insbesondere bevorzugt sind Triblockcopolymere der folgenden Formeln

R^{x}[EOₓ₁][PO_{y3}][EOₓ₂]OR^{x'}

R^{x}[EOₓ₁][PO_{y1}]Y-A-Y[PO_{y2}][EOₓ₂]R^{x'}

R^{x}[PO_{y1}][EOₓ₃][PO_{y2}]OR^{x'}

Hierbei wird die Einheit [PO_{y1}]A[PO_{y2}] als ein PO-Block betrachtet. In den Formeln stehen R^{x} und R^{x'} unabhängig voneinander für Wasserstoff oder C₁-C₁₀-Alkyl und EO, PO haben die zuvor genannten Bedeutungen. Die Indices x1 und x2 weisen unabhängig voneinander einen Wert im Bereich von 2 bis 100, insbesondere 4 bis 50 auf. Die Indices y1 und y2 weisen unabhängig voneinander einen Wert im Bereich von 2 bis 100, insbesondere 4 bis 50 auf. Der Index y3 steht typischerweise für einen Wert von 2 bis 160, insbesondere für einen Wert von 4 bis 100 und speziell für 10 bis 80. Der Index x3 steht typischerweise für einen Wert von 4 bis 200, insbesondere für einen Wert von 10 bis 100 und speziell für 10 bis 80. A steht für C₄-C₁₀-Alkandiyl oder C₅-C₁₀-Cycloalkandiyl. Y steht für Sauerstoff oder für einen Rest NR, worin R für Wasserstoff, C₁-C₄-Alkyl oder eine Gruppe der Formel R^{x}[EOₓ₁][PO_{y1}] steht. Das zahlenmittlere Molekulargewicht der EO/PO-Copolymere liegt vorzugsweise im Bereich von 300 bis 10000 Dalton, insbesondere im Bereich von 500 bis 5000 Dalton. Der Anteil der EO-Wiederholungseinheiten liegt typischerweise im Bereich von 10 bis 90 Gew.-%, insbesondere im Bereich von 20 bis 80 Gew.-% und der Anteil der PO-Wiederholungseinheiten im Bereich von 10 bis 90 Gew.-%, insbesondere im Bereich von 20 bis 80 Gew.-%, jeweils bezogen auf die Gesamtgewicht des EO/PO-Copolymers.

Erfindungsgemäß umfasst die nichtionische oberflächenaktive Substanz wenigstens ein oberflächenaktive Substanzen aus der Gruppe der Poly-C₂-C₃-alkoxylate, bevorzugt aus den Gruppen c.10, c.11 und/oder c.15.und/oder ein Gemisch eines oder mehrerer, z. B. 1 oder 2, Poly-C₂-C₃-alkoxylate mit einem EO/PO-Copolymer, und insbesondere einem EO/PO-Blockcopolymer.

Die erfindungsgemäßen Wirkstoffkonzentrate enthalten weiterhin als Komponente D1 wenigstens ein aprotisch polares organisches Lösungsmittel, das eine Mischbarkeit mit Wasser bei 25 °C und 1 bar von wenigstens 50 g/L, insbesondere wenigstens 100 g/L aufweist und insbesondere mit Wasser vollständig mischbar ist, und das ausgewählt ist unter:
- Amide, N-C₁-C₄-Alkylamide und N,N-C₁-C₄-Dialkylamide aliphatischer Carbonsäuren mit 1 bis 12, insbesondere 1 bis 6 C-Atomen, insbesondere die Amide, N-C₁-C₂-Alkylamide und N,N-C₁-C₂-Dialkylamide der Ameisensäure, der Essigsäure, der Propionsäure, der Valeriansäure und der Capronsäure wie Formamid, Dimethylformamid, Acetamid, Propionamid, N,N-Dimethylacetamid, Dimethylpropionamid und Dimethylvaleramid;
- Sulfolan und Dimethylsulfoxid, sowie
- 5-, 6- und 7-gliedrige Lactame, die am Stickstoffatom eine N-C₁-C₄-Alkylgruppe, insbesondere eine Methylgruppe aufweisen, beispielsweise N-C₁-C₄-Alkylpyrrolidone wie N-Methylpyrrolidon, N-Ethylpyrrolidon, oder N-C₁-C₄-Alkylvalerolactame wie N-Methylvalerolactam.

Bevorzugte aprotisch polare Lösungsmittel sind die vorgenannten Amide und die N,N-C₁-C₄-Dialkylamide aliphatischer C₁-C₆-Carbonsäuren, insbesondere die Amide und Dimethylamide dieser Carbonsäuren, speziell der Ameisensäure, der Essigsäure, der Propionsäure und der Valeriansäure, weiterhin N-C₁-C₄-Alkylpyrrolidone, speziell N-Methylpyrrolidon, N-C₁-C₄-Alkylvalerolactame, speziell N-Methylvalerolactam, sowie Dimethylsulfoxid und Gemische davon.

Insbesondere umfasst das aprotisch polare organische Lösungsmittel zu wenigstens 80 Gew.-%, bezogen auf die Gesamtmenge an aprotisch polarem organischen Lösungsmittel in der Formulierung, eines der vorgenannten bevorzugten aprotisch polaren Lösungsmittel, insbesondere Dimethylsulfoxid und/oder N-Methylpyrrolidon.

Weiterhin umfasst das erfindungsgemäße, nicht-wässrige Wirkstoffkonzentrat wenigstens ein organisches Lösungsmittel, das bei 25 °C und 1 bar eine Löslichkeit in Wasser von weniger als 5 g/l, insbesondere weniger als 1 g/l aufweist. Hierzu zählen insbesondere Kohlenwasserstofflösungsmittel und C₁-C₁₀-Alkylester von Fettsäuren. Bei den Kohlenwasserstofflösungsmitteln handelt es sich um einen bei Raumtemperatur flüssigen Kohlenwasserstoff oder ein flüssiges Kohlenwasserstoffgemisch, wie es typischerweise zur Herstellung von emulgierbaren Wirkstoffkonzentraten eingesetzt wird. Geeignete Kohlenwasserstoffe sind Alkane mit vorzugsweise 6 bis 14 C-Atomen, Cycloalkane, die gegebenenfalls 1, 2 ,3 oder 4 C₁-C₄-Alkylgruppen aufweisen und die vorzugsweise insgesamt 6 bis 14 C-Atome haben, aromatische Kohlenwasserstoffe wie Benzol, Naphthalin, Mono-, Di- und Tri-C₁-C₄-alkyl substituiertes Benzol, insbesondere Toluol, Xylole, Mesitylen, Cumol, sowie C₁-C₄-Alkyl substituiertes Naphthalin sowie Gemische der vorgenannten Kohlenwasserstoffe. Bevorzugt sind insbesondere Kohlenwasserstoffe und Kohlenwasserstoffgemische mit einem Gehalt an aromatischen Kohlenwasserstoffen von wenigstens 50 Gew.-% und insbesondere wenigstens 80 Gew.-%. Bevorzugt sind weiterhin Kohlenwasserstoffe und Kohlenwasserstoffgemische, deren Siedepunkt bzw. deren minimale Siedetemperatur nach ASTM D86 wenigstens 150 °C, insbesondere 180 °C und speziell wenigstens 200 °C beträgt. Derartige Kohlenwasserstoffe und Kohlenwasserstoffgemische sind dem Fachmann geläufig und im Handel erhältlich, beispielsweise unter den Bezeichnungen Shellsol® A der Shell AG und unter der Bezeichnung Solvesso®, beispielsweise unter den Bezeichnungen Solvesso® 100, Solvesso®150, Solvesso®150 ND, Solvesso®200, Solvesso®200 ND und Solvesso®200 S.

Zu den Alkylestern von Fettsäuren zählen insbesondere die C₁-C₆-Alkylester und speziell die Methylester aliphatischer gesättigter oder ungesättigter C₆-C₂₀-Monocarbonsäuren, insbesondere die Ester der Capronsäure, der Önanthsäure, der Caprylsäure, der Pelargonsäure, der Caprinsäure, der Undecansäure, der Laurinsäure, der Myristinsäure, der Palmitinsäure, der Stearinsäure, der Ölsäure oder der Palmitoleinsäure, sowie Gemische von Fettsäure-C₁-C₆-alkylestern, insbesondere -methylestern, wie sie durch Umesterung nativer Triglyceride mit C₁-C₆-Alkanolen, speziell Methanol, erhalten werden, z. B. Sojaölmethylester, Rapsölmethylester, Palmitinsäuremethylester, Stearinsäuremethylester und Ölsäuremethylester und Gemischen davon.

Bevorzugt sind Kohlenwasserstofflösungsmittel.

In den erfindungsgemäßen, nicht-wässrigen Wirkstoffkonzentraten beträgt das Gewichtsverhältnis von aprotisch polarem organischem Lösungsmittel zu Kohlenwasserstofflösungsmittel vorzugsweise 1 : 10 bis 10 : 1, insbesondere 1 : 5 bis 5 : 1.

Die Gesamtmenge an organischem Lösungsmittel, d. h. aprotisch polarem organischen Lösungsmittel und Kohlenwasserstofflösungsmittel liegt typischerweise im Bereich von 200 bis 800 g/L und insbesondere im Bereich von 300 bis 600 g/L.

Weiterhin können die erfindungsgemäßen, nicht-wässrigen Wirkstoffkonzentrate übliche Bestandteile enthalten, wie sie typischerweise in Emulsionskonzentraten herbizider Wirkstoffe verwendet werden. Hierzu zählen beispielsweise Antischaummittel und Konservierungsmittel. Vorzugsweise wird der Anteil dieser Bestandteile 5 Gew.-% und insbesondere 1 Gew.-%, bezogen auf das Gesamtgewicht des nicht-wässrigen Wirkstoffkonzentrats nicht überschreiten.

Die Herstellung der erfindungsgemäßen, nicht-wässrigen Wirkstoffkonzentrate kann in Analogie zur Herstellung konventioneller emulgierbarer Konzentrate erfolgen. Vorzugsweise wird man zunächst eine Lösung der wenigstens einen, 4-Benzoyl-substituierten Pyrazolverbindung I in wenigstens einem Teil des aprotisch polaren Lösungsmittels herstellen. Gegebenenfalls erfolgt die Herstellung dieser Lösung unter Erwärmen, wobei man jedoch vorzugsweise Temperaturen von 80 °C nicht überschreitet. In der Regel erfolgt die Herstellung der Lösung bei Umgebungstemperatur oder im Bereich von 10 bis 50 °C. Zu der so erhaltenen Lösung gibt man dann die weiteren Bestandteile der Formulierung, wobei man Dimethenamid bzw. Dimethenamid P in gelöster Form oder als Feststoff zugeben kann. Häufig wird man so vorgehen, dass man nacheinander das Kohlenwasserstofflösungsmittel, Dimethenamid bzw. Dimethenamid P, die oberflächenaktiven Substanzen und gegebenenfalls weitere Bestandteile nacheinander zu der Lösung der Verbindung der Formel I in dem aprotisch polaren organischen Lösungsmittel gibt und die so erhaltene Mischung mit geeigneten Vorrichtungen homogenisiert, beispielsweise mittels geeigneter Rührer, Dissolver und dergleichen, bis eine klare homogene Mischung erhalten wird. Die Zugabe von Kohlenwasserstofflösungsmittel, Dimethenamid bzw. Dimethenamid P, der oberflächenaktiven Substanzen und gegebenenfalls weiterer Bestandteile sowie das Homogenisieren erfolgt typischerweise bei Umgebungstemperatur oder im Bereich von 10 bis 50 °C. Das so erhaltene Wirkstoffkonzentrat kann dann in üblicher Weise konfektioniert und verpackt werden.

Ein zweiter Gegenstand der vorliegenden Erfindung betrifft ein wässriges Wirkstoffkonzentrat gemäß obiger Definition.

In den erfindungsgemäßen, wässrigen Wirkstoffkonzentraten liegen die Wirkstoffe der Formel I und Dimethenamid in disperser Form, d. h. in Form fein verteilter Partikel vor. Der Begriff Partikel umfasst hier sowohl feste Wirkstoffpartikel als auch flüssige Wirkstofftröpfchen. Ohne an eine Theorie gebunden zu sein, nimmt man an, dass der überwiegende Anteil des wenigstens einen Wirkstoffs der Formel I in Form fester Wirkstoffpartikel vorliegt, wohingegen der überwiegende Anteil des Dimethenamids vermutlich in Form öliger Tröpfchen vorliegt. Die Teilchengröße der Wirkstoffpartikel (feste Wirkstoffpartikel sowie Tröpfchen) wird typischerweise 50 µm, insbesondere 20 µm und speziell 10 µm nicht überschreiten (sog. d₉₀-Wert, d. h. derjenige Wert, den höchstens 10 Gew.-% der im Konzentrat enthaltenen Wirkstoffteilchen überschreiten). Die gewichtsmittlere Teilchengröße (sog. d₅₀-Wert) liegt typischerweise im Bereich von 0,1 bis 10 µm und insbesondere im Bereich von 0,5 bis 5 µm. Die hier angegebenen Werte beziehen sich auf die mittels quasi elastische Lichtstreuung an verdünnten wässrigen Proben der Wirkstoffkonzentrate bestimmten Werte (Verdünnungsrate 1 : 20 bis 1 : 200).

Erfindungsgemäß enthalten die wässrigen Wirkstoffkonzentrate Gemische wenigstens einer nichtionische oberflächenaktive Substanz mit wenigstens einer anionischen oberflächenaktiven Substanz. Die Gesamtkonzentration an oberflächenaktiven Substanzen, d. h. die Konzentration an nichtionischer oberflächenaktiver Substanz plus der gegebenenfalls vorhandenen anionischen oberflächenaktiven Substanzen, liegt erfindungsgemäß im Bereich von 10 bis 200 g/L, insbesondere im Bereich von 15 bis 150 g/L und speziell im Bereich von 20 bis 100 g/L. In den erfindungsgemäßen wässrigen Wirkstoffkonzentraten beträgt das Gewichtsverhältnis von nichtionischer oberflächenaktiver Substanz zu anionischer oberflächenaktiver Substanz vorzugsweise 100 : 1 bis 10 : 1, insbesondere 50 : 1 bis 5 : 1.

Geeignete nichtionische oberflächenaktive Substanzen sind grundsätzlich alle zuvor bei den nicht-wässrigen Wirkstoffkonzentraten genannten nichtionischen oberflächenaktiven Substanzen, vorzugsweise nichtionische oberflächenaktive Substanzen aus der Gruppe der Poly(C₂-C₃-alkoxylate), beispielsweise Substanzen der Gruppen c.10. bis c.16, insbesondere nichtionische oberflächenaktive Substanzen der Gruppen c.10, c.11 und c.12. Hierunter besonders bevorzugt sind die Poly(ethoxylat-co-propoxylate) der Gruppen c.10. und c.11. Derartige Verbindungen lassen sich durch die allgemeine Formel (III) beschreiben

R-O-[(A-0)ₓ;(E-O)_{y}]R' (III)

worin
- R: für C₁₀-C₂₂-Alkyl, C₈-C₂₂-Alkylphenyl, Mono-, Di- oder Tristyryl steht,
- R': für Wasserstoff, C₁-C₁₀-Alkyl, Benzyl, Formyl oder C₁-C₁₀-Alkylcarbonyl, insbesondere Wasserstoff, steht,
- A: für CH(CH₃)CH₂ steht,
- E: für CH₂CH₂ steht,
- x: für eine Zahl im Bereich von 1 bis 30 insbesondere 1 bis 10 und
- y: für eine Zahl im Bereich von 2 bis 50 insbesondere 2 bis 30 stehen.

Als nichtionische oberflächenaktive Substanzen sind insbesondere auch Ethylenoxid-propylenoxid-Copolymere geeignet, wie sie auch im Zusammenhang mit den nicht-wässrigen Wirkstoffkonzentraten bereits genannt wurden, insbesondere die dort genannten Triblockcopolymere

In einer bevorzugten Ausführungsform umfasst die nichtionische oberflächenaktive Substanz wenigstens eine nichtionische oberflächenaktive Substanz der Gruppen c.10 bis c.16. insbesondere der Gruppen c.10. und/oder c.11. und speziell wenigstens eine nichtionische oberflächenaktive Substanz der allgemeinen Formel III, sowie gegebenenfalls wenigstens ein EO/PO-Copolymer, speziell ein EO/PO-Blockcopolymer der zuvor beschriebenen Art. In dieser Ausführungsform liegt das Gewichtsverhältnis von der wenigstens einen oberflächenaktiven Substanz der Gruppen c.10. bis c.16. zu dem/den EO/PO-Copolymer(en) typischerweise im Bereich von 100 : 1 bis 1 : 1 und speziell im Bereich von 50 : 1 bis 5 : 1.

Daneben enthält das erfindungsgemäße wässrige Wirkstoffkonzentrat auch eine oder mehrere anionische oberflächenaktive Substanzen. Geeignete oberflächenaktive Substanzen sind grundsätzlich all diejenigen, die zuvor im Zusammenhang mit den nicht-wässrigen Wirkstoffkonzentraten genannt wurden, insbesondere anionische oberflächenaktive Substanzen der Gruppen c.1 bis c.9. und speziell anionische oberflächenaktive Substanzen der Gruppe c.9.

Die erfindungsgemäßen wässrigen Wirkstoffkonzentrate können zusätzlich noch weitere Stoffe enthalten, die nicht im unmittelbaren Zusammenhang mit der Zielsetzung der Zusammensetzungen stehen, aber ihre Anwendbarkeit und/oder praktischen Eigenschaften verbessern. Beispiele hierfür sind insbesondere
- Viskositäts-Regler (Verdicker)
- Konservierungsmittel,
- Antischaummittel,
- Mittel zur Einstellung des pH-Werts,
- Frostschutzmittel.

Entsprechende Stoffe sind dem Fachmann geläufig. Die Gesamtmenge derartiger Stoffe wird in der Regel 10 Gew.-% (= ca.100 g/L), bezogen auf das Wirkstoffkonzentrat nicht überschreiten und liegt typischerweise im Bereich von 0.1 bis 10 Gew.-% (= 1 bis 100 g/L), bezogen auf das Gesamtgewicht der Wirkstoffkonzentrats.

Zu den Viskosität verändernden Additiven (Andicker) zählen insbesondere Verbindungen, die bekanntermaßen wässrigen Formulierungen ein pseudoplastisches Fließverhalten verleihen, d. h. hohe Viskosität im Ruhezustand und niedrige Viskosität im bewegten Zustand. Geeignet sind grundsätzlich alle für diesen Zweck in wässrigen Wirkstoffkonzentraten eingesetzte Verbindungen. Zu nennen sind beispielsweise anorganische Substanzen wie Bentonite oder Attapulgite (z. B. Attaclay^{®} Firma Engelhardt), und organische Substanzen wie Polysaccharide und Heteropolysaccharide wie Xanthan Gum^{®} (Kelzan^{®} der Fa. Kelco), Rhodopol^{®} 23 (Rhone Poulenc) oder Veegum^{®} (Firma R.T. Vanderbilt) zu nennen, wobei Xanthan-Gum^{®} bevorzugt verwendet wird. Die Menge der die Viskosität verändernden Additive beträgt häufig 0,1 bis 5 Gew.-%, bezogen auf das Gesamtgewicht des Wirkstoffkonzentrats.

Als geeignete Antischaummittel kommen beispielsweise für diesen Zweck bekannte Silikonemulsionen (Silikon^{®} SRE, Firma Wacker oder Rhodorsil^{®} der Firma Rhodia), langkettige Alkohole, Fettsäuren, Entschäumer vom Typ wässriger Wachsdispersionen, feste Entschäumer (sog. Compounds), fluororganische Verbindungen und deren Gemische in Betracht. Die Menge an Antischaummittel beträgt typischerweise 0,1 bis 3 Gew.-%, gerechnet als Schaumaktive Substanz und bezogen auf das Gesamtgewicht des Wirkstoffkonzentrats.

Beispiele für Konservierungsmittel sind solche auf Basis von Isothiazolonen, beispielsweise Proxel^{®} der Fa. ICI oder Acticide^{®} RS der Fa. Thor Chemie oder Kathon^{®} MK der Firma Rohm & Haas. Die Menge an Konservierungsmitteln beträgt, sofern vorhanden, typischerweise 0,05 bis 0,5 Gew.-%, bezogen auf das Gesamtgewicht des Wirkstoffkonzentrats.

Geeignete Frostschutzmittel sind flüssige Alkanole wie Methanol, Ethanol, Isopropanol, n-Butanol, Polyole, z. B. Ethylenglycol, Propylenglycol oder Glycerin. Die Menge an Frostschutzmitteln beträgt, sofern vorhanden, in der Regel 1 bis 10 Gew.-%, bezogen auf das Gesamtgewicht des Wirkstoffkonzentrats.

Die Herstellung der erfindungsgemäßen wässrigen Wirkstoffkonzentrate kann in Analogie zu bekannten Verfahren zur Herstellung von Suspensionskonzentraten oder Suspo-Emulsionskonzentraten, die wenigstens zwei unterschiedliche Wirkstoffe enthalten, erfolgen.

Hierzu wird man in der Regel so vorgehen, dass man eine wässrige Suspension des wenigstens einen Wirkstoffs der Formel I und getrennt hiervon eine wässrige Suspension oder Emulsion von Dimethenamid herstellt und die beiden Suspensionen bzw. die Suspension mit der Emulsion zum erfindungsgemäßen wässrigen Wirkstoffkonzentrat vereinigt. Die Herstellung der Suspensionen der Verbindungen I kann ebenso wie die Herstellung der Suspensionen bzw. Emulsionen des Dimethenamids in Analogie zur Herstellung wässriger Suspensionskonzentrate von organischen Pflanzenschutzwirkstoffen erfolgen.

Beispielsweise kann man eine erste wässrige Suspension des wenigstens einen Wirkstoffs der Formel I herstellen, indem man zunächst eine wässrige Aufschlämmung des wenigstens einen Wirkstoffs der Formel I herstellt und anschließend auf die gewünschte Teilchengröße vermahlt. In der Regel enthält die wässrige Aufschlämmung einen Teil der im Konzentrat enthaltenen oberflächenaktiven Substanzen, beispielsweise ein Ethylenoxid-Propylenoxid-Copolymer und gegebenenfalls eine anionische oberflächenaktive Substanz, sowie gegebenenfalls Entschäumer und gegebenenfalls einen Teil oder die Gesamtmenge des Frostschutzmittels. Der so erhaltenen wässrigen Suspension des wenigstens einen Wirkstoffs I können dann Wasser und weitere Bestandteile, beispielsweise die Restmenge an Frostschutzmittel, Verdicker und Biozid zugesetzt werden, wobei die Zugabe der Hilfsstoffe typischerweise in Form einer wässrigen Lösung erfolgt.

Die Herstellung der wässrigen Emulsion bzw. Suspension des Dimethenamids kann in an sich bekannter Weise in Analogie zur Herstellung von wässrigen Konzentraten des Dimethenamids erfolgen. Häufig wird man so vorgehen, dass man zunächst eine wässrige Lösung, die wenigstens einen Teil der oberflächenaktiven Substanzen, insbesondere wenigstens eine oberflächenaktive Substanz der Formel III enthält, vorlegt und hierin Dimethenamid, gegebenenfalls unter Erwärmen suspendiert bzw. emulgiert. Die wässrige Vorlage kann zusätzlich einen Teil oder die Gesamtmenge der weiteren Bestandteile des wässrigen Wirkstoffkonzentrats, beispielsweise Verdicker, Biozid, einen Teil des Frostschutzmittels und gegebenenfalls Entschäumer enthalten.

Anschließend werden die Suspension des wenigstens einen Wirkstoffs der Formel I mit der wässrigen Suspension bzw. Emulsion des Dimethenamids unter Durchmischung vereinigt, beispielsweise unter Rühren, wobei man die fertige Formulierung enthält. Selbstverständlich ist es möglich, im Anschluss daran, einen Teil der optionalen Zusätze, vorzugsweise in Form einer wässrigen Lösung, zuzugeben.

Die erfindungsgemäßen nicht-wässrigen Wirkstoffkonzentrate, ebenso wie die erfindungsgemäßen wässrigen Wirkstoffkonzentrate, eignen sich in an sich bekannter Weise zur Bekämpfung von unerwünschtem Pflanzenwuchs. Die erfindungsgemäßen Wirkstoffkonzentrate eignen sich insbesondere zur Bekämpfung von unerwünschtem Pflanzenwuchs auf Nichtkulturflächen sehr gut, besonders bei hohen Aufwandmengen. In Kulturen wie Getreide, z. B. Weizen, Roggen, Gerste, Hafer, Hirse und Triticale, sowie Mais wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die erfindungsgemäßen Wirkstoffkonzentrate noch in einer weiteren Zahl von Kulturpflanzen zur Beseitigung unerwünschter Pflanzen eingesetzt werden.

Darüber hinaus können die Wirkstoffkonzentrate auch in Kulturen, die durch Züchtung einschließlich gentechnischer Methoden gegen die Wirkung von Herbiziden tolerant sind, verwandt werden.

Die Applikation der Wirkstoffkonzentrate erfolgt in der Regel in Form einer wässrigen Spritzbrühe. Hierzu werden die erfindungsgemäßen Wirkstoffkonzentrate, abhängig von der Aufwandmenge, auf ein Vielfaches ihres Volumens, beispielsweise auf das 10-bis 10000-fache, insbesondere auf das 20- bis 1000-fache mit Wasser verdünnt. Die Wirkstoffkonzentration in der Spritzbrühe liegt dann typischerweise im Bereich von 10 mg/l bis 10 g/l.

Die Applikation kann im Vorauflauf-, im Nachauflaufverfahren oder zusammen mit dem Saatgut einer Kulturpflanze erfolgen. Es besteht auch die Möglichkeit, die in den Wirkstoffkonzentraten enthaltenen Wirkstoffe der Formeln I und Dimethenamid bzw. Dimethenamid P dadurch zu applizieren, dass man unter Verwendung der erfindungsgemäßen Wirkstoffkonzentrate Saatgut einer Kulturpflanze mit einer wässrigen Verdünnung der Wirkstoffkonzentrate behandelt und das so behandelte Saatgut ausbringt. Sind die in den erfindungsgemäßen Wirkstoffkonzentraten enthaltenen Wirkstoffe für gewisse Kulturpflanzen weniger verträglich, so können Ausbringungstechniken angewandt werden, bei welchen die unter Verwendung der Wirkstoffkonzentrate hergestellten Applikationsformen mit Hilfe der Spritzgeräte so gespritzt werden, dass die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen, bezogen auf die Gesamtmenge an Wirkstoff betragen je nach Bekämpfungsziel, Jahreszeit, Zielpflanzen und Wachstumsstadium 0,001 bis 3,0, vorzugsweise 0,01 bis 1,0 kg/ha aktive Substanz (a. S.).

Zur Verbreiterung des Wirkungsspektrums und zur Erzielung synergistischer Effekte können die Wirkstoffkonzentrate vor dem Ausbringen mit zahlreichen Vertretern anderer herbizider oder wachstumsregulierender Wirkstoffgruppen gemischt und gemeinsam ausgebracht werden, beispielsweise im Tank-Mix. Beispielsweise kommen als Mischungspartner 1,2,4-Thiadiazole, 1,3,4-Thiadiazole, Amide, Aminophosphorsäure und deren Derivate, Aminotriazole, Anilide, (Het)-Aryloxyalkansäure und deren Derivate, Benzoesäure und deren Derivate, Benzothiadiazinone, 2-Aroyl-1,3-cyclohexandione, 2-Hetaroyl-1,3-cyclohexandione, Hetaryl-Aryl-Ketone, Benzylisoxazolidinone, Meta-CF₃-phenylderivate, Carbamate, Chinolincarbonsäure und deren Derivate, Chloracetanilide, Cyclohexenonoximether-Derivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- oder Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Wirkstoffkonzentrate vor dem Ausbringen auch noch mit weiteren Pflanzenschutzmitteln zu vermischen und gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die nachfolgenden Beispiele dienen der weiteren Erläuterung der Erfindung und sind nicht einschränkend zu verstehen.

Einsatzmaterialien:
- Topramezone (Wirkstoff der Formel I, worin R¹ und R⁵ jeweils für Methyl stehen, R² für 4,5-Dihydroisoxazol-3-yl steht, R³ Methylsulfonyl bedeutet, R⁴ und R⁶ für Wasserstoff stehen);
- Dimethenamid P
- Emulgator 1: Gemisch aus Calciumdodecylbenzolsulfonat, Castoroil-Ethoxylat, EO/PO-Triblockcopolymer und dem Phosphatester eines Fettalkohols mit einem Gehalt an oberflächenaktiven Substanzen von ≥ 85 Gew.-%.
- Emulgator 2: EO/PO-Triblockcopolymer mit einem Molekulargewicht von 6500 und einem Propylenoxid-Anteil von 50 Gew.-%.
- Emulgator 3: Natriumsalz eines Phenolsulfonsäure-Formaldehyd-Kondensationsprodukts
- Emulgator 4: Gemisch von Poly(ethoxylat-co-propoxylaten) von Tristyrylphenol
- Verdicker: Xanthan-Gum
- Entschäumer: Handelsübliche Polydimethylsiloxan-Füllstoff-Emulsion (Wacker Silikon SRE-PFL) (Aktivgehalt 20 Gew.-%)
- Mikrobiozid: Formulierung, enthaltend Gemisch aus 1,2-Benzisothiazolin-3-on und 2-Methyl-4-isothiazolin-3-on, Aktivgehalt 5 Gew.-% (Aktizide MBS der Thor Chemie GmbH).
- Kohlenwasserstoff-Lösungsmittel: Aromatisches Kohlenwasserstoffgemisch mit einem Gehalt aromatischer Verbindungen von wenigstens 99 Gew.-% und einer Mindestsiedetemperatur, bestimmt nach ASTM 86 bis 99, im Bereich von 235 bis 248 °C und einer maximalen Siedetemperatur im Bereich von 290 bis 305 °C (Solvesso® 200 der Exxon Mobil)

### Beispiel 1: Herstellung eines nicht-wässrigen Wirkstoffkonzentrats

In einem Rührkessel legte man 219 g N-Methylpyrrolidon vor und gab hierzu 32 g Topramezone und rührte, bis man eine klare, homogene Mischung erhielt. Hierzu gab man nacheinander unter Rühren 219 g Kohlenwasserstoff-Lösungsmittel, 32 g Topramezone und 112 g Emulgator 1 und rührte, bis die Mischung homogen war. Die erhaltene Mischung war eine rötlich-braune Flüssigkeit, die 538 g/L Dimethenamid P und etwa 32 g/L Topramezone enthielt.

Die Dichte, bestimmt bei 20 °C, lag bei etwa 1,11 bis 1,12 g/cm³. Die Viskosität, bestimmt mit einem Rotationsviskosimeter nach der OECD Testvorschrift 114 lag bei etwa 20 bis 35 mPa·s. Die Probe wies nach zweiwöchiger Lagerung bei 54 °C keine sichtbaren Veränderungen auf. Die Schaumhöhe einer 0,3 gew.-%igen Verdünnung, bestimmt nach Ross-Miles (ASTM-D 1173 53) betrug nicht mehr als 30 mm. Die Emulsionsstabilität nach CIPAC MT lag bei 36,3.

### Beispiel 2: Herstellung eines nicht-wässrigen Wirkstoffkonzentrats

In einem Rührkessel legte man 219 g Dimethylsulfoxid vor und gab hierzu 32 g Topramezone und rührte, bis man eine klare, homogene Mischung erhielt. Hierzu gab man nacheinander unter Rühren 219 g Kohlenwasserstoff-Lösungsmittel, 32 g Topramezone und 112 g Emulgator 1 und rührte, bis die Mischung homogen war. Die erhaltene Mischung war eine rötlich-braune Flüssigkeit, die 538 g/L Dimethenamid P und etwa 32 g/L Topramezone enthielt.

Die Dichte, bestimmt bei 20 °C, lag bei etwa 1,11 bis 1,12 g/cm³. Die Viskosität, bestimmt mit einem Rotationsviskosimeter nach der OECD Testvorschrift 114 lag bei etwa 20 bis 35 mPa·s. Die Probe wies nach zweiwöchiger Lagerung bei 54 °C keine sichtbaren Veränderungen auf. Die Schaumhöhe einer 0,3 gew.-%igen Verdünnung, bestimmt nach Ross-Miles (ASTM-D 1173 53) betrug nicht mehr als 30 mm.

### Beispiel 3: Herstellung eines nicht-wässrigen Wirkstoffkonzentrats

In einem Rührkessel legte man 219 g N-Methylpyrrolidon vor und gab hierzu 32 g Topramezone und rührte, bis man eine klare, homogene Mischung erhielt. Hierzu gab man nacheinander unter Rühren 219 g Kohlenwasserstoff-Lösungsmittel, 32 g Topramezone und 112 g eines Gemischs aus Calcium-Dodecylbenzolsulfonat und Emulgator 5 im Gewichtsverhältnis 1:1 und rührte, bis die Mischung homogen war. Die erhaltene Mischung war eine rötlich-braune Flüssigkeit, die 538 g/L Dimethenamid P und etwa 32 g/L Topramezone enthielt.

Die Dichte, bestimmt bei 20 °C, lag bei etwa 1,11 bis 1,12 g/cm³. Die Viskosität, bestimmt mit einem Rotationsviskosimeter nach der OECD Testvorschrift 114 lag bei etwa 20 bis 35 mPa·s.

### Beispiel 4: Herstellung eines erfindungsgemäßen wässrigen Wirkstoffkonzentrats

1. In einem Rührgefäß legte man 400 g entmineralisiertes Wasser vor und gab hierzu nacheinander 60 g 1,2 Propylenglykol, 20 g Emulgator 3 und 166,7 g einer 18 gew.-%igen, wässrigen Lösung des Emulgators 2. Man rührte bis eine homogene klare Lösung erhalten wurde und gab dann hierzu nacheinander 343,9 g technisches Topramezone mit einem Topramezone-Gehalt von 97,7 Gew.-% und 1 g Entschäumer. Man kühlte die so erhaltene Suspension auf etwa 15 °C und leitete sie dann durch eine Rotor-/Statormühle und anschließend unter Kühlung durch eine Kugelmühle, bis die gewünschte Teilchengrößenverteilung erreicht war. Man erhielt auf diese Weise eine wässrige Topramezone-Suspension, worin 80 Gew.-% der Teilchen einen Durchmesser unterhalb 2 µm aufwiesen.
2. In einem Rührgefäß legte man 10 g 1,2 Propylenglykol und 119,4 g entmineralisiertes Wasser vor und gab dann unter Rühren nacheinander 3 g Verdicker und anschließend 2 g des Mikrobiozids. Anschließend gab man die so erhaltene Lösung unter Rühren zu der in Schritt 1 erhaltenen Suspension und gab hiernach weitere 4 g des Entschäumers unter Rühren zu. Auf diese Weise erhielt man eine wässrige Suspension, die etwa 336 g/L Topramezone enthielt und die eine Viskosität, bestimmt nach OECD 114, von etwa 60 bis 100 mPa·s aufwies. Die Teilchengrößenverteilung war durch einen d₉₀-Wert von ≤ 3,5 µm und einem d₅₀-Wert von ≤ 1,3 µm charakterisiert.
3. Zu 285,7 g entmineralisiertem Wasser gab man unter Rühren 44,4 g 1,2-Propylenglykol, 44,4 g des Emulgators 3 und 66,6 g einer 2 gew.-%igen, wässrigen Lösung des Verdickers, die 1,6 Gew.-% des Biozids enthielt. Zu dieser Lösung gab man unter Rühren bei 23 °C 561 g Dimethenamid P und rührte so lange, bis man eine stabile Emulsion erhielt. Zu der so erhaltenen Emulsion gab man anschließend 107,6 g der in Schritt 2 erhaltenen Suspension und rührte weitere 10 Minuten.

Auf diese Weise erhielt man eine wässrige Suspo-Emulsion, die einen Gehalt an Dimethenamid-P von etwa 538 g und einen Gehalt an Topramezone von etwa 32 g/L aufwies. Die Dichte lag bei etwa 1,11 g/cm³. Die Viskosität, bestimmt mit einem Rotationsviskosimeter nach der OECD-Testvorschrift 114 lag bei etwa 70 bis 90 mPa·s. Der d₉₀-Wert lag unterhalb 7 µm und der d₅₀-Wert lag unterhalb 1,5 µm. Der pH-Wert einer etwa 1 gew.-%igen Verdünnung in entmineralisiertem Wasser lag im Bereich von etwa 2,5 bis 4,5.

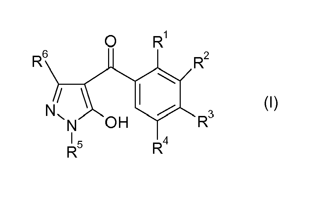

## Patentansprüche

1. Nicht-wässriges Wirkstoffkonzentrat, enthaltend
a) 10 bis 100 g/L mindestens einer 4-Benzoyl-substituierten Pyrazolverbindung der Formel I 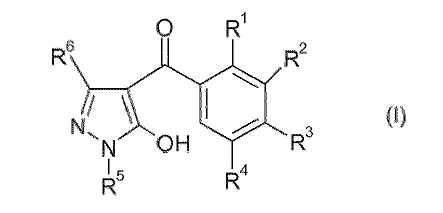 worin
R¹ und R⁵ jeweils Methyl bedeuten, R² für 4,5-Dihydroisoxazol-3-yl steht, R³ Methylsulfonyl bedeutet und R⁴ und R⁶ Wasserstoff bedeutet,
oder eines seiner landwirtschaftlich verwendbaren Salze,
b) 200 bis 700 g/L 2-Chlor-N-(2,4-dimethyl-3-thienyl)-N-(2-methoxy-1-methyl-ethyl)acetamid, und
c) 10 bis 200 g/L mindestens einer oberflächenaktiven Substanz S, die ausgewählt ist unter einem Gemisch wenigstens einer anionischen oberflächenaktiven Substanz und wenigstens einer nichtionischen oberflächenaktiven Substanz,
wobei die anionische oberflächenaktive Substanz ausgewählt ist unter Verbindungen, die wenigstens eine SO₃-Gruppe oder eine PO₄-Gruppe und wenigstens einen aliphatischen Kohlenwasserstoffrest mit 8 bis 22 C-Atomen oder einen araliphatischen Kohlenwasserstoffrest mit 10 bis 24 C-Atomen aufweisen, und die nichtionische oberflächenaktive Substanz als Hauptbestandteil wenigstens eine Poly-C₂-C₃-alkylenglykolether-Verbindung umfasst
wobei die Bestandteile a), b) und c) in einem organischen Lösungsmittelgemisch gelöst vorliegen, das zu wenigstens 95 Gew.-%, bezogen auf das Lösungsmittelgemisch, aus
d1) wenigstens einem aprotisch polaren organischen Lösungsmittel, das eine Mischbarkeit mit Wasser bei 25 °C und 1 bar von wenigstens 50 g/l aufweist, und das ausgewählt ist unter Dimethylsulfoxid, Sulfolan, den Amiden, N-C₁-C₄-Alkylamiden und N,N-Di-(C₁-C₄-alkyl)amiden aliphatischer Monocarbonsäuren mit 1 bis 12 C-Atomen, N-C₁-C₄-Alkyllactamen und deren Gemischen;
d2) wenigstens einem organischen Lösungsmittel, das in Wasser bei 25 °C und 1 bar eine Löslichkeit von weniger als 5 g/l aufweist, besteht.

2. Wirkstoffkonzentrat nach Anspruch 1, worin das organische Lösungsmittelgemisch 200 bis 800 g/L der Formulierung ausmacht.

3. Wirkstoffkonzentrat nach Anspruch 1 oder 2, worin das aprotisch polare, organische Lösungsmittel ausgewählt ist unter Dimethylsulfoxid, N-Methylpyrrolidon, N-Ethylpyrrolidon und deren Gemischen.

4. Wirkstoffkonzentrat nach einem der vorhergehenden Ansprüche, worin das Gewichtsverhältnis von aprotisch polarem Lösungsmittel und Kohlenwasserstoff-Lösungsmittel im Bereich von 5 : 1 bis 1 : 5 liegt.

5. Wässriges Wirkstoffkonzentrat, enthaltend
a) 10 bis 100 g/L mindestens einer 4-Benzoyl-substituierten Pyrazolverbindung der Formel I wie in Anspruch 1 definiert,
b) 200 bis 700 g/L 2-Chlor-N-(2,4-dimethyl-3-thienyl)-N-(2-methoxy-1-methyl-ethyl)acetamid, und
c) 10 bis 200 g/L mindestens einer oberflächenaktiven Substanz S, die ausgewählt ist unter Gemischen wenigstens einer nichtionischen oberflächenaktiven Substanz mit wenigstens einer anionischen oberflächenaktiven Substanz,
wobei die Bestandteile a) und b) in einem wässrigen Verdünnungsmittel in disperser Form vorliegen.

6. Wirkstoffkonzentrat nach Anspruch 5, wobei die nichtionische oberflächenaktive Substanz als Hauptbestandteil eine Verbindung der Formel III enthält
R-O-[(A-O)ₓ;(E-O)_{y}]R' (III)
worin
R für C₁₀-C₂₂-Alkyl, C₈-C₂₂-Alkylphenyl, Mono-, Di- oder Tristyryl steht,
R' für Wasserstoff, C₁-C₁₀-Alkyl, Benzyl, Formyl oder C₁-C₁₀-Alkylcarbonyl steht,
A für CH(CH₃)CH₂ steht,
E für CH₂CH₂ steht,
x für eine Zahl im Bereich von 1 bis 30 und
y für eine Zahl im Bereich von 2 bis 50 stehen.

7. Wirkstoffkonzentrat nach Anspruch 6, wobei die nichtionische oberflächenaktive Substanz zusätzlich wenigstens ein Ethylenoxid-Propylenoxid-Blockcopolymer umfasst.

8. Wirkstoffkonzentrat nach einem der vorhergehenden Ansprüche, worin das Gewichtsverhältnis von Verbindung I zu 2-Chlor-N-(2,4-dimethyl-3-thienyl)-N-(2-methoxy-1-methylethyl)acetamid im Bereich von 1:5 bis 1:50 liegt.

9. Wirkstoffkonzentrat nach einem der vorhergehenden Ansprüche, enthaltend 2-Chlor-N-(2,4-dimethyl-3-thienyl)-N-(2-methoxy-1-methylethyl)acetamid in Form seines (S)-Enantiomers oder in Form einer nicht-racemischen Mischung der beiden Enantiomere mit einem Enantiomeren-Überschuss des S-Enantiomers von wenigstens 80 %.

10. Verwendung eines Wirkstoffkonzentrats nach einem der Ansprüche 1 bis 9 zur Bekämpfung unerwünschten Pflanzenwuchses.

11. Verfahren zur Bekämpfung unerwünschten Pflanzenwuchses, **dadurch gekennzeichnet, dass** man eine wässrige Spritzbrühe durch Verdünnen eines Wirkstoffkonzentrats nach einem der Ansprüche 1 bis 9 herstellt und diese auf Pflanzen, deren Samen und/oder deren Lebensraum einwirken lässt.

12. Verfahren nach Anspruch 11, **dadurch gekennzeichnet, dass** man die Blätter der unerwünschten Pflanzen mit der wässrigen Spritzbrühe behandelt.

## Claims

1. A non-aqueous active compound concentrate, comprising
a) from 10 to 100 g/l of at least one 4-benzoyl-substituted pyrazole compound of the formula I 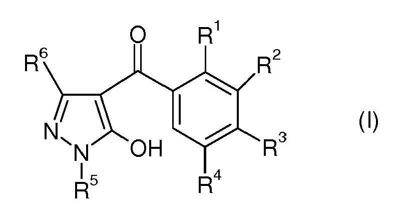 in which
R¹ and R⁵ are each methyl, R² is 4,5-dihydroisoxazol-3-yl, R³ is methylsulfonyl and R⁴ and R⁶ are hydrogen,
or one of its agriculturally useful salts,
b) from 200 to 700 g/l of 2-chloro-N-(2,4-dimethyl-3-thienyl)-N-(2-methoxy-1-methylethyl)acetamide, and
c) from 10 to 200 g/l of at least one surfactant S selected from a mixture of at least one anionic surfactant and at least one nonionic surfactant,
where the anionic surfactant is selected from the group consisting of compounds comprising at least one SO₃ group or one PO₄ group and at least one aliphatic hydrocarbon radical having 8 to 22 carbon atoms or an araliphatic hydrocarbon radical having 10 to 24 carbon atoms, and the nonionic surfactant comprises, as main component, at least one poly-C₂-C₃-alkylene glycol ether compound
where the components a), b) and c) are present dissolved in a mixture of organic solvents consisting to at least 95% by weight, based on the solvent mixture, of
d1) at least one aprotic polar organic solvent having a miscibility with water at 25°C and 1 bar of at least 50 g/l, and which is selected from the group consisting of dimethyl sulfoxide, sulfolane, the amides, N-C₁-C₄-alkylamides and N,N-di(C₁-C₄-alkyl)amides of aliphatic monocarboxylic acids having 1 to 12 carbon atoms, N-C₁-C₄-alkyllactams and mixtures thereof;
d2) at least one organic solvent having a solubility in water at 25°C and 1 bar of less than 5 g/l.

2. The active compound concentrate according to claim 1 in which the mixture of organic solvents comprises from 200 to 800 g/l of the formulation.

3. The active compound concentrate according to claim 1 or 2 in which the aprotic polar organic solvent is selected from the group consisting of dimethyl sulfoxide, N-methylpyrrolidone, N-ethylpyrrolidone and mixtures thereof.

4. The active compound concentrate according to any of the preceding claims in which the weight ratio of aprotic polar solvent to hydrocarbon solvent is in the range of from 5:1 to 1:5.

5. An aqueous active compound concentrate, comprising
a) from 10 to 100 g/l of at least one 4-benzoyl-substituted pyrazole compound of the formula I as defined in claim 1,
b) from 200 to 700 g/l of 2-chloro-N-(2,4-dimethyl-3-thienyl)-N-(2-methoxy-1-methylethyl)acetamide, and
c) from 10 to 200 g/l of at least one surfactant S selected from mixtures of at least one nonionic surfactant with at least one anionic surfactant,
where the components a) and b) are present in disperse form in an aqueous diluent.

6. The active compound concentrate according to claim 5 where the nonionic surfactant comprises, as main component, a compound of the formula III
R-O-[(A-O)ₓ; (E-0)_{y}]R' (III)
in which
R is C₁₀-C₂₂-alkyl, C₈-C₂₂-alkylphenyl, mono-, di-or tristyryl,
R' is hydrogen, C₁-C₁₀-alkyl, benzyl, formyl or C₁-C₁₀-alkylcarbonyl,
A is CH(CH₃)CH₂,
E is CH₂CH₂,
x is a number in the range from 1 to 30 and
y is a number in the range from 2 to 50.

7. The active compound concentrate according to claim 6 where the nonionic surfactant additionally comprises at least one ethylene oxide/propylene oxide block copolymer.

8. The active compound concentrate according to any of the preceding claims wherein the weight ratio of compound I to 2-chloro-N-(2,4-dimethyl-3-thienyl)-N-(2-methoxy-1-methylethyl)acetamide is in the range of from 1:5 to 1:50.

9. The active compound concentrate according to any of the preceding claims, comprising 2-chloro-N-(2,4-dimethyl-3-thienyl)-N-(2-methoxy-1-methylethyl)acetamide in the form of its (S)-enantiomers or in the form of a non-racemic mixture of the two enantiomers having an enantiomeric excess of the S-enantiomer of at least 80%.

10. The use of an active compound concentrate according to any of claims 1 to 9 for controlling unwanted vegetation.

11. A method for controlling unwanted vegetation which comprises preparing an aqueous spray liquor by diluting an active compound concentrate according to any of claims 1 to 9 and allowing the spray liquor to act on plants, their seeds and/or their habitat.

12. The method according to claim 11 wherein the leaves of the unwanted plants are treated with the aqueous spray liquor.

## Revendications

1. Concentré non aqueux de principe actif, contenant :
a) 10 à 100 g/l d'au moins un composé de pyrazole à substitution 4-benzoyle de formule I 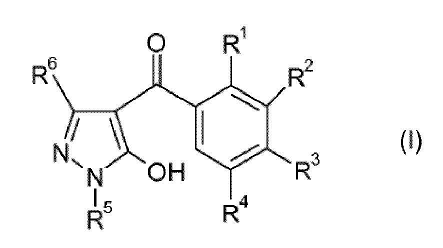 dans laquelle
R¹ et R⁵ signifient chacun méthyle, R² représente 4,5-dihydroisoxazol-3-yle, R³ signifie méthylsulfonyle, et R⁴ et R⁶ signifient hydrogène,
ou un de ses sels utilisables en agriculture,
b) 200 à 700 g/l de 2-chloro-N-(2,4-diméthyl-3-thiényl)-N-(2-méthoxy-1-méthyl-éthyl)acétamide, et
c) 10 à 200 g/l d'au moins une substance tensioactive S, qui est choisie parmi un mélange d'au moins une substance tensioactive anionique et d'au moins une substance tensioactive non ionique,
la substance tensioactive anionique étant choisie parmi les composés qui comprennent au moins un groupe SO₃ ou un groupe PO₄ et au moins un radical hydrocarboné aliphatique de 8 à 22 atomes C ou un radical hydrocarboné araliphatique de 10 à 24 atomes C, et la substance tensioactive non ionique comprenant en tant que constituant principal au moins un composé d'éther de polyalkylène glycol en C₂-C₃,
les constituants a), b) et c) étant dissous dans un mélange de solvants organiques qui est constitué à hauteur d'au moins 95 % en poids, par rapport au mélange de solvants, par
d1) au moins un solvant organique aprotique polaire, qui présente une miscibilité avec l'eau à 25°C et 1 bar d'au moins 50 g/l et qui est choisi parmi le diméthylsulfoxyde, le sulfolane, les amides, les N-alkylamides en C₁-C₄ et les N,N-di(alkyle en C₁-C₄) amides d'acides monocarboxyliques aliphatiques de 1 à 12 atomes C, les N-alkyllactames en C₁-C₄ et leurs mélanges ;
d2) au moins un solvant organique, qui présente dans l'eau à 25°C et 1 bar une solubilité de moins de 5 g/1.

2. Concentré de principe actif selon la revendication 1, dans lequel le mélange de solvants organiques représente 200 à 800 g/l de la formulation.

3. Concentré de principe actif selon la revendication 1 ou 2, dans lequel le solvant organique aprotique polaire est choisi parmi le diméthylsulfoxyde, la N-méthylpyrrolidone, la N-éthylpyrrolidone et leurs mélanges.

4. Concentré de principe actif selon l'une quelconque des revendications précédentes, dans lequel le rapport en poids entre le solvant aprotique polaire et le solvant hydrocarboné est dans la plage allant de 5:1 à 1:5.

5. Concentré aqueux de principe actif, contenant :
a) 10 à 100 g/l d'au moins un composé de pyrazole à substitution 4-benzoyle de formule I tel que défini dans la revendication 1,
b) 200 à 700 g/l de 2-chloro-N-(2,4-diméthyl-3-thiényl)-N-(2-méthoxy-1-méthyl-éthyl)acétamide et
c) 10 à 200 g/l d'au moins une substance tensioactive S, qui est choisie parmi les mélanges d'au moins une substance tensioactive non ionique avec au moins une substance tensioactive anionique,
les constituants a) et b) se présentant sous forme dispersée dans un diluant aqueux.

6. Concentré de principe actif selon la revendication 5, dans lequel la substance tensioactive non ionique contient en tant que constituant principal un composé de formule III
R-O-[(A-O)x; (E-O)_{y}]R' (III)
dans laquelle
R représente alkyle en C₁₀-C₂₂, alkylphényle en C₈-C₂₂, mono-, di- ou tristyryle,
R' représente hydrogène, alkyle en C₁-C₁₀, benzyle, formyle ou alkylcarbonyle en C₁-C₁₀,
A représente CH(CH₃)CH₂,
E représente CH₂CH₂,
x représente un nombre dans la plage allant de 1 à 30, et
y représente un nombre dans la plage allant de 2 à 50.

7. Concentré de principe actif selon la revendication 6, dans lequel la substance tensioactive non ionique comprend en outre au moins un copolymère séquencé d'oxyde d'éthylène-oxyde de propylène.

8. Concentré de principe actif selon l'une quelconque des revendications précédentes, dans lequel le rapport en poids entre le composé I et le 2-chloro-N-(2,4-diméthyl-3-thiényl)-N-(2-méthoxy-1-méthyl-éthyl)acétamide est dans la plage allant de 1:5 à 1:50.

9. Concentré de principe actif selon l'une quelconque des revendications précédentes, contenant le 2-chloro-N-(2,4-diméthyl-3-thiényl)-N-(2-méthoxy-1-méthyl-éthyl)acétamide sous la forme de son énantiomère (S) ou sous la forme d'un mélange non racémique des deux énantiomères avec un excès énantiomérique de l'énantiomère S d'au moins 80 %.

10. Utilisation d'un concentré de principe actif selon l'une quelconque des revendications 1 à 9 pour lutter contre la végétation indésirable.

11. Procédé de lutte contre la végétation indésirable, **caractérisé en ce qu'**une bouillie de pulvérisation aqueuse est fabriquée par dilution d'un concentré de principe actif selon l'une quelconque des revendications 1 à 9, et celle-ci est laissée agir sur des plantes, leurs graines et/ou leur habitat.

12. Procédé selon la revendication 11, **caractérisé en ce que** les feuilles des plantes indésirables sont traitées avec la bouillie de pulvérisation aqueuse.
